# EUROPEAN PATENT APPLICATION

(11) **EP 3 312 601 A1**
(43) Date of publication of application: **25.04.2018**
(21) Application number: 17203637.8
(22) Date of filing: 04.02.2015
(51) Int. Cl.: G01N 27/416, G01N 27/30, G01N 27/407, G01N 27/333, G01N 33/18

(54) **HYDROGEN PHOSPHATE ION SENSOR COMPRISING A MIXED OXIDE CATALYST OF RUTHENIUM OXIDE AND TANTALUM OXIDE**

(30) Priority: 12.02.2014 JP 2014024895; 11.11.2014 JP 2014229330
(62) Divisional of application: 15749488.1
(71) Applicant: The Doshisha, Kyoto 602-8580 (JP)
(72) Inventor: Morimitsu, Masatsugu, Kyoto (JP)
(74) Representative: Piotrowicz, Pawel Jan Andrzej

(57) **Abstract**

An ion sensor for quantifying hydrogen phosphate ions in water includes a sensing electrode using an ion sensor catalyst. The ion sensor catalyst includes a mixed oxide of ruthenium oxide and tantalum oxide.

## Description

### TECHNICAL FIELD

The present invention relates to an ion sensor catalyst for quantifying hydrogen phosphate ions in water, an ion sensor for quantifying hydrogen phosphate ions using the ion sensor catalyst, and a quantification method for quantifying the concentration of hydrogen phosphate ion and/or the concentration of total phosphorus using the ion sensor catalyst.

### BACKGROUND ART

Phosphorus exists in rocks and is also contained in any animals and plants and their excrement. Moreover, phosphorus is also contained in fertilizers, agrichemicals, and synthetic detergents, and it is often the case that phosphorus increase in environmental water (river water, seawater, lake and marsh water, or the like) is derived from incorporation of household wastewater, night soil, domestic wastewater, industrial wastewater, agricultural wastewater, or stock farming drainage. For example, phosphorus is barely removed by typical wastewater treatment (up to secondary treatment), so that effluents from night soil treatment facilities or sewage treatment facilities might cause phosphorus increase in environmental water. Phosphorus is one of the nutrients for organisms, but once phosphorus concentration increases, eutrophication in rivers, lakes, marshes, seas, fishery water, etc., is promoted, resulting in environmental issues such as a massive bloom of plankton or a red tide. Accordingly, in view of environmental conservation, it is important and necessary to measure phosphorus concentration in environmental water, industrial wastewater, agricultural wastewater, household wastewater, domestic wastewater, stock farming drainage, treated wastewater, aquaculture fishery water, night soil, effluents from sewage treatment facilities, etc. On the other hand, in biological systems, phosphorus exists as various forms of phosphoric acid ester, including phosphate ion, DNA, and RNA. Phosphorus compounds are important controlling factors in a biological system's environment; ATP, which is essential for energy metabolism of organisms, and DNA are composed of nucleotides, which have phosphates included in a part of their molecules, and the biomass of organisms is significantly limited by the amount of phosphates obtained from the environment. Furthermore, the blood concentration of phosphorus is also important, and the reference range of the adult plasma concentration of inorganic PO₄ is from 2.5 mg/dL to 4.5 mg/dL: concentration less than the reference range is referred to as hypophosphatemia where muscle weakness, respiratory failure, heart failure, a spasm, or a coma might be caused, and in contrast, concentration greater than the reference range is referred to as hyperphosphatemia, which is caused by chronic renal failure, hypoparathyroidism, or the like.

Here, phosphorus compounds are classified into inorganic and organic forms, and inorganic phosphorus is further divided into orthophosphate-phosphorus and polyphosphate-phosphorus. The state of orthophosphate-phosphorus in water changes depending on pH, and main forms in various pH ranges are as follows: phosphorus acid H₃PO₃ (where the pH is 2 or lower), dihydrogen phosphate ion H₂PO₄⁻ (where the pH is from 2 to 7), hydrogen phosphate ion HPO₄²⁻ (where the pH is from 7 to 12), and phosphate ion PO₄³⁻ (where the pH is 12 or higher). The polyphosphate-phosphorus includes pyrophosphate (P₂O₇⁴⁻), which changes into hydrogen phosphate ions through hydrolysis or suchlike. Moreover, in addition to the foregoing, the organic phosphorus also includes phosphorus-containing organic compounds such as esters and phospholipids. Examples of the organic phosphorus include tripolyphosphate included in synthetic detergents and sewage treatment agents.

In Japan, standard values and acceptable limits for phosphorus concentration are defined by Environmental Quality Standards Related to the Preservation of the Living Environment and effluent standards according to Water Pollution Control Act. Here, the phosphorus concentration refers to the concentration of phosphorus generally defined as "total phosphorus", typically represented in units of mg/L and indicating the weight of phosphorus contained in 1L of sample water, as obtained by a predetermined method after dissolving a phosphorus compound present in the sample water to be measured for concentration into orthophosphate-phosphorus by a strong acid or another oxidizer or by hydrolysis or suchlike. In a method for measuring the concentration of such total phosphorus, organic phosphorus or polyphosphate-phosphorus in sample water is entirely decomposed into orthophosphate-phosphorus by potassium peroxydisulfate or by nitric acid and sulfuric acid, and thereafter, colored by a method called a molybdenum blue method, so that the concentration of total phosphorus can be obtained on the basis of the absorbance of the colored product. This method requires the steps of: chemically treating the sample water, reacting phosphate ions and molybdenum acid, and generating molybdenum blue for coloring by adding a reducing agent such as ascorbic acid; and measuring absorbance using an absorptiometer. Furthermore, the concentration of only the orthophosphate-phosphorus can be determined by the aforementioned method, i.e., a combination of the molybdenum blue method and absorptiometry, without including the chemical treatment as described above, but adjusting the pH of the sample water to the ph range where phosphate ions can be present, or the concentration of only the orthophosphate-phosphorus can be determined by a method in which phosphate ions are isolated by ion chromatography using an ion exchange resin, and the concentration is obtained through electrical conductivity measurement or ultraviolet absorption measurement. The above-described quantification of total phosphorus and phosphate ions is also defined by JIS K 0102. In addition, Patent Documents 1 and 2 also disclose methods for measuringphosphate ion concentration, andbothmethods are similar to the aforementioned method defined by JIS in that absorbance or transmittance is measured after phosphate ions in sample water assume a color or are colored in reaction with a plurality of reagents. Note that as described earlier, the environmental quality standard and the effluent standard define the concentrations for "total phosphorus", and there is no environmental quality standard nor effluent standard for orthophosphate-phosphorus. Moreover, in these methods, resulting from the pH of the sample water being adjusted, orthophosphate-phosphorus exist as PO₄³⁻, and both organic phosphorus and orthophosphate-phosphorus (inorganic phosphorus) are measured in the form of PO₄³⁻. Accordingly, as for orthophosphate-phosphorus in the environment, concentration is not obtained for each existence form, and further, such method for determining concentration for each existence form has not yet been developed.

Here, in Japan, concerning the effluent standards for phosphorus, Water Pollution Control Law lists organic phosphorus compounds as hazardous items in Effluent Standards, with the acceptable limit being set as 1 mg/L; further, the concentration of total phosphorus where specified ground-permeating water is to be deemed to contain a hazardous substance is set as 0.1 mg/L, and ground permeation of any specified ground-permeating water under such condition is considered to be against the law. Moreover, concerning living environment items, Effluent Standards indicates an acceptable phosphorus content limit of 16 mg/L (daily average: 8 mg/L) for the concentration of total phosphorus. Note that the criteria for the living environment items apply to effluents poured into lakes, marshes, and ocean areas specified by the Ministry of the Environment and also into public water bodies communicating with such. Furthermore, although tentative, there are standards indicating 30 mg/L (daily average: 24 mg/L) for livestock agriculture and 40 mg/L (daily average: 10 mg/L) for phosphorus compound manufacturing. On the other hand, as for the environmental quality standards, "Environmental Quality Standards Concerning the Protection of Human Health" do not include stipulations for phosphorus, but "Environmental Water Quality Standards Concerning the Conservation of the Living Environment" regulate standard values concerning lakes and marshes, and the concentration of total phosphorus is set as 0.1 mg/L or lower. This concentration of total phosphorus, when represented in the molar concentration of phosphorus (mmol/L), falls within the range of from 10⁻³ mmol/L to 10 mmol/L. Note that the environmental quality standards are "standards desired to be maintained for the purpose of protecting human health and conserving the living environment", and therefore, sets lower values than the effluent standards. As described above, at present, quantification of total phosphorus or phosphate ions is performed by either the method in which sample water is chemically treated to transform phosphorus entirely into phosphate ions, and then color and spectroscopically analyze the phosphate ions or by the method in which phosphate ions are isolated through ion chromatography, and electrical conductivity or ultraviolet absorption of the solution containing the phosphate ions is measured, and therefore, the quantification process is complicated and takes time to finally obtain the concentration after the treatment of the sample water. Moreover, in these methods, phosphorus is entirely transformed into PO₄³⁻, and therefore, there is no means of individually measuring the concentration of other types of phosphorus, more specifically, orthophosphate-phosphorus.

On the other hand, for example, Patent Document 3 discloses a hydrogen phosphate ion sensor and quantification method in which, unlike in the methods described above, hydrogen phosphate ions are brought into contact with at least one compound selected from among aromatic acids and/or aromatic derivatives, and quantified in a solution thereof through ultraviolet-visible absorptiometry, a fluorescence-phosphorescence emission method, or nuclear magnetic resonance spectroscopy. The hydrogen phosphate ion sensor and quantification method is intended to be applied to a sensing system for detecting not only the aforementioned quantification of the concentration of phosphorus in the environment but also hydrogen phosphate ion in a living organism because a signal transduction system within the living organism controls various information transmissions via phosphate groups in phosphoproteins and phospholipids. However, the invention disclosed in Patent Document 3 requires large-sized analytical equipment for spectroscopy or nuclear magnetic resonance, which takes time to obtain measurements, and also requires analytical reagents such as aromatic acids and aromatic derivatives. That is, the conventional techniques described so far are methods incapable of directly and solely detecting a target ion regardless whether the ion is phosphate ion (PO₄³⁻) or hydrogen phosphate ion (HPO₄²⁻).

On the other hand, methods for electrochemically detecting phosphorus have been studied. For example, Patent Document 4 discloses a method for measuring the concentrations of a fluorine compound and a phosphate compound in which electrical conductivity of sample water, fluoride ion concentration measured at a fluoride ion electrode, and ultrasonic propagation or pH measurements are compared to a pre-generated data table, thereby indirectly determining the concentration of the phosphate compound. Moreover, Patent Document 5 discloses a method for qualitatively and quantitatively analyzing phosphate ions in which a reduced-form mediator is generated by allowing an oxidized-form mediator to coexist when phosphate ions are reacted with a substrate to be oxidized in reaction with phosphate ions in the presence of an oxidation-reduction enzyme that catalyzes a reaction to generate the oxidized substrate, and a current generated by further oxidizing the reduced-form mediator is measured. Furthermore, Non-Patent Document 1 discloses a method in which phosphate ion concentration is indirectly quantified on the basis of a current flowing when phosphate ions are reacted in a multi-phase enzymatic reaction and the resultant hydrogen peroxide (H₂O₂) is oxidized on an electrode.

Further, Non-Patent Documents 2 through 4 disclose methods for electrochemically detecting hydrogen phosphate ion concentration, rather than phosphate ion concentration. In these methods, oxidation current or impedance at an electrode, which changes depending on hydrogen phosphate ion concentration in sample water, is measured, and hydrogen phosphate ions are quantified on the basis of the obtained oxidation current or impedance. In Non-Patent Documents 2 through 4, a composite oxide (an oxide solid solution of two or more types of metallic elements dissolved at the atomic level along with oxygen), such as perovskite oxide, spinel oxide, or pyrochlore oxide, is used as a catalyst for a sensing electrode for electrochemically detecting hydrogen phosphate ions, and oxidation current or impedance at the sensing electrode is measured in sample water containing hydrogen phosphate ions; these non-patent documents report the relationship between the logarithm of the hydrogen phosphate ions and the detected oxidation current or impedance.

On the other hand, as a result of studying electrochemical reaction in sample water containing hydrogen phosphate ions where iridium dioxide (IrO₂) was used as the material of a sensing electrode, the inventor of the present invention has already demonstrated that oxidation current is generated on the oxide because of hydrogen phosphate ions, and that hydrogen phosphate ion concentration and oxidation current are in proportion, and the present inventor has also demonstrated that oxidation current density (i.e., current per unit area of the sensing electrode) at that time is approximately 100 to 1000 times as much as values disclosed in Non-Patent Documents 2 through 4 for the same concentration range and that time to obtain stable oxidation current (detection time) is shorter; however, in the case of the sensing electrode using iridium dioxide as a catalyst, the range of concentration that is in proportion to oxidation current is narrow (from 1 mmol/L to 10 mmol/L), resulting in limited quantifiable concentration.

### Prior Art Documents

### Patent Documents

Patent Document 1: Japanese Laid-Open Patent Publication No. 2006-84451
Patent Document 2: Japanese Laid-Open Patent Publication No. 2005-99014
Patent Document 3: Japanese Laid-Open Patent Publication No. 2007-40760
Patent Document 4: Japanese Laid-Open Patent Publication No. 2005-265501
Patent Document 5: Re-publication of PCT International Publication No. WO2005/73399

### Non-Patent Documents

Non-Patent Document 1: Toshio Yao, MizuhoWada, and Tamotsu Wasa, BUNSEKI KAGAKU, Vol. 42, pp. 397-400 (1993)
Non-Patent Document 2: Youichi Shimizu, Satoko Takase, and Koji Araki, Annual Research Report of The Salt Science Research Foundation, No. 0605 (2008)
Non-Patent Document 3: S. Takase, T. Matsumoto, Y. Shimizu, Electrochemistry, Vol. 78, No. 2, pp. 150-152 (2010)
Non-Patent Document 4: Y. Shimizu, A. Ishikawa, K. Iseki, S. Takase, J. Electrochem. Soc., Vol. 147, No. 10, pp. 3931-3934 (2010).

### DISCLOSURE OF THE INVENTION

### PROBLEMS TO BE SOLVED BY THE INVENTION

As described earlier, it is important and necessary to measure the concentration of phosphorus present either in the environment or in a living organism, and particularly, if hydrogen phosphate ions can be quantified directly and electrochemically using a catalyst, such a method can be applied to either case. However, up to now, there is no sensor nor quantification method put to practical use for directly and electrochemically quantifying not only hydrogen phosphate ions but also both forms of phosphorus, either organic or inorganic, through electrode reaction without using an oxidized-form mediator or a reduced-form mediator. If hydrogen phosphate ions can be quantified electrochemically, the concentration of total phosphorus can be obtained directly on the basis of a current value where hydrogen phosphate ions are obtained by simply subjecting sample water to oxidation treatment and pH adjustment, and thereafter, electrochemically oxidized on a catalyst. Moreover, phosphorus concentration in a biological sample can also be determined in a similar manner without large-sized equipment such as a spectral device or a nuclear magnetic resonator. In addition, an ion sensor provided with a sensing electrode using such a catalyst can be miniaturized to a portable size, and makes it easy to perform on-the-spot measurement and continuous monitoring in a wide range of applications such as environmental measurement, medical practice, and various analytical tasks.

While there is some demand for development of an ion sensor capable of electrochemically detecting hydrogen phosphate ions and determining concentration as described above, conventional techniques for measuring hydrogen phosphate ion concentration in water have the following problems:
(1) For example, in the case of measuring oxidation current density for hydrogen phosphate ions in sample water using a composite oxide as a catalyst, as described in Non-Patent Documents 2 through 4, if the catalyst allows detection of low oxidation current density and quantification over a wide range of concentration, detection sensitivity is about 1/1000 compared to the case where iridium dioxide is used as a catalyst.
(2) In addition, there is no proportional relationship between concentration and oxidation current density, and therefore, no calibration curve can be obtained to linearly approximate the relationship.
(3) On the other hand, as described earlier, iridium dioxide allows measurement over only a narrow range of concentration.
(4) In addition, for any of the aforementioned conventional methods for electrochemically detecting hydrogen phosphate ions, the catalyst still has only provided low detection sensitivity, and there is no catalyst that is capable of providing high detection sensitivity over a wide range and makes it possible to determine concentration accurately from high to low ranges with concentration and oxidation current density being in proportion.

The present invention solves the above problems by providing an inexpensive ion sensor catalyst capable of detecting hydrogen phosphateionsin water and determining hydrogen phosphate ion concentration on the basis of oxidation current density for the ions, the ion sensor catalyst being characterized by providing higher detection sensitivity than conventional ion sensor catalysts, being capable of maintaining high detection sensitivity over a wide range of hydrogen phosphate ion concentration, achieving a proportional relationship between concentration and oxidation current density, so that the concentration can be determined with accuracy regardless of the concentration range, and allowing oxidation current to reach a steady-state value in a short time, so that time to determine current density is short, resulting in shortened time to quantify hydrogen phosphate ions, and stable response to hydrogen phosphate ion oxidation can be made repeatedly; the present invention also provides an ion sensor for measuring hydrogen phosphate ions and a quantification method, the ion sensor is capable of quantifying hydrogen phosphate ions in a short time with high detection sensitivity and accuracy over a wide concentration range by using the catalyst, while being provided in a portable size, mass-produced at low cost, and at the same time, being superior in measurement stability and repeatability, and the quantification method uses the sensor and achieves highly sensitive and repeatable quantification of total phosphorus and hydrogen phosphate ions.

### SOLUTION TO THE PROBLEMS

The present inventor has completed the present invention after having carried out various studies to solve the aforementioned problems, and found that the problems can be solved by a catalyst including a mixed oxide of ruthenium oxide (RuO₂) and tantalum oxide (Ta₂O₅), an ion sensor using the catalyst as a sensing electrode, and a quantification method.

More specifically, to solve the above problems, the present invention provides the ion sensor catalyst, the ion sensor using the same, and the quantification method with the following features. The ion sensor catalyst of the present invention is a catalyst for electrochemically oxidizing hydrogen phosphate ions in water, including a mixed oxide of ruthenium oxide and tantalum oxide. These features render it possible to achieve the following effects:
(1) The mixed oxide of ruthenium oxide and tantalum oxide can selectively exhibit high catalytic activity for electrochemical oxidation of hydrogen phosphate ions in water over a wide range of hydrogen phosphate ion concentration while achieving high oxidation current density. This allows the ion sensor catalyst of the present invention to detect hydrogen phosphate ions with higher detection sensitivity than other catalysts and determine the concentration of the ion.
(2) Hydrogen phosphate ion concentration and oxidation current density are in a proportional relationship, so that the concentration can be determined with accuracy regardless of the concentration range; oxidation current reaches a steady-state value in a short time, so that time to determine current density is short, resulting in shortened time to quantify hydrogen phosphate ions, and stable response to hydrogen phosphate ion oxidation can be made repeatedly, resulting in superior measurement stability and repeatability.
(3) The ion sensor catalyst of the present invention has higher detection sensitivity than composite oxides and iridium dioxide in the conventional art, and is capable of quantifying a significantly wide concentration range compared to iridium dioxide, resulting in high accuracy and superior reliability.
(4) Hydrogen phosphate ion quantification can be performed with high catalytic activity and detection sensitivity over a wide concentration range using an inexpensive material compared to the conventional art because ruthenium costs about 1/10 of iridium and tantalum costs about 1/10 of ruthenium, so that superior resource saving, practicality, and measurement stability can be achieved.
(5) The catalyst of the present invention does not oxidize other negative or positive ions, such as dihydrogen phosphate ion or chloride ion, and therefore, can selectively detect only the hydrogen phosphate ion and determine the concentration thereof, so that superior measurement certainty and reliability can be achieved.

Here, the ion sensor catalyst of the present invention (hereinafter, the catalyst of the present invention) can be synthesized and utilized in a manner below. The catalyst of the present invention is intended to oxidize hydrogen phosphate ions and detect the resultant oxidation current, and is required to be connected to a conductive portion for outputting the detected current to the exterior, and for example, if the catalyst of the present invention is formed on a conductive substrate, the catalyst can be utilized as a sensing electrode on the conductive substrate serving as a conductive portion for external current output. Moreover, the conductive portion may be formed as a part of an insulative substrate, and the catalyst of the present invention may be formed on a part of the conductive portion as a sensing electrode. Note that to electrochemically oxidize hydrogen phosphate ions, besides the sensing electrode formed by the catalyst of the present invention, it is necessary to provide another electrode (i.e., a counter electrode) which causes at least some type of reduction. Moreover, in the case where hydrogen phosphate ions are oxidized while the sensing electrode is regulated at a predetermined potential, a reference electrode is required for regulating the potential of the sensing electrode.

In the case where the conductive substrate is used, the sensing electrode and the counter electrode, alone or in combination with the reference electrode, are required to be provided on the same conductive substrate so as not to be conductive to each other, but in the case where the insulative substrate is used, the conductive portion with the catalyst of the present invention formed thereon and the counter electrode, or the counter electrode and the reference electrode, are electrically insulated from each other, so that the sensing electrode and the counter electrode, alone or in combination with the reference electrode, can be formed on the same insulative substrate. For the conductive substrate and the conductive portion, various conductive materials, such as metal and alloy, various carbon materials (including conductive diamond, graphene, fullerene, etc.), conductive plastic, compounds such as conductive oxide, and metal pastes containing silver or suchlike, can be used, but these are not limiting. For the insulative substrate, various insulative oxides (such as alumina), carbides, and nitrides, as well as diamond, DLC (diamond-like carbon), silicon, and organic materials such as plastic and resin can be used, but these are not limiting. The conductive portion, the conductive substrate, and the insulative substrate can be provided in various forms such as plate, mesh, bar, sheet, tube, line, spiral, perforated plate, porous form, sphere, cage, and three-dimensional porous form with bonded particles. Moreover, carbon fibers, carbon nanotubes, or arrays of regularly disposed carbon fibers or carbon nanotubes can also be used as the conductive portion and the conductive substrate. In addition, the catalyst of the present invention or a catalyst support carrying the catalyst of the present invention on its surface may be carried on or mixed with any of the aforementioned various forms of conductive portions or conductive substrates, thereby creatively increasing the surface area per unit mass of the catalyst of the present invention.

The methods that can be used to form the catalyst of the present invention either on the conductive substrate or on the conductive portion of the insulative substrate include thermal decomposition in which the conductive substrate or the conductive portion is thermally treated at a predetermined temperature after a precursor solution containing ruthenium and tantalum is applied thereto, and also includes methods that directly form the catalyst through various types of physical or chemical vapor deposition such as sputtering and CVD and various other methods in which the catalyst of the present invention is formed on the conductive substrate or the conductive portion through any of various processes such as application, baking, bonding, welding, or adsorption of a catalyst synthesized in advance by, for example, a sol-gel process or another precursor calcining method. Here, an example method where the catalyst of the present invention is formed on a titanium plate, which is an example of the conductive substrate, through thermal decomposition will be described. For example, a precursor solution in which ruthenium and tantalum in an arbitrary form such as inorganic compounds, organic compounds, ions, or complexes are dissolved is applied to a titanium plate and thermally decomposed at 200°C to 600°C, thereby forming a mixed oxide composed of ruthenium oxide and tantalum oxide on the titanium plate. For example, as a precursor solution, a butanol solution in which ruthenium(III) chloride trihydrate and tantalum(V) chloride are dissolved is applied to the titanium plate and thermally decomposed. At this time, the range of a preferred molar ratio of ruthenium to tantalum can be appropriately selected by a thermal decomposition temperature or suchlike. However, as the proportion of ruthenium in the total amount of ruthenium and tantalum falls below 1%, the conductivity of the catalyst of the present invention itself becomes susceptible to decrease, so that oxidation current to be detected tends to be lower, whereas as the proportion of tantalum in the total amount of ruthenium and tantalum falls below 1%, high catalytic activity exerted on hydrogen phosphate ions by mixing ruthenium oxide and tantalum oxide tends to be lost readily, and neither of the above cases is preferable.

In the catalyst of the present invention obtained by thermal decomposition for the aforementioned temperature range, tantalum oxide is amorphous, whereas ruthenium oxide is amorphous, crystalline, or in a combined state of amorphous and crystalline depending on the thermal decomposition temperature. For the catalyst of the present invention, ruthenium oxide may take any of these crystal structures. The difference in crystal structure can be relatively determined on the basis of the intensity of a diffraction line for ruthenium oxide obtained by X-ray diffractometry; the intensity of the diffraction line decreases as the crystallinity of ruthenium oxide lessens, and the diffraction line disappears when the crystallinity is lost completely. Note that the above example has been described with respect to the case where tantalum oxide is amorphous, but tantalum oxide may be crystalline. The above-described example of thermal decomposition is not limited by the use of the butanol solvent, the molar ratio of ruthenium and tantalum, the thermal decomposition temperature range related thereto, these conditions are merely illustrative, and the catalyst of the present invention may be obtained by any other methods in addition to the above, so long as a mixed oxide of ruthenium oxide and tantalum oxide can be obtained.

In the ion sensor catalyst of the present invention, the mixed oxide preferably contains ruthenium and tantalum in a molar ratio of from 30:70 to 80:20. This feature renders it possible to further achieve the following effect:
(1) As a result of the molar ratio of ruthenium and tantalum in the mixed oxide being from 30:70 to 80:20, particularly high catalytic activity on hydrogen phosphate ions can be achieved over a wide concentration range. Here, as the proportion of ruthenium falls below 30%, the conductivity of the mixed oxide lessens, and oxidation current density for the hydrogen phosphate ions tends to be lower, whereas as the proportion of ruthenium exceeds 80%, the effect achieved by mixing with tantalum oxide becomes harder to be achieved, and oxidation current density for the hydrogen phosphate ions tends to be lower, and neither of the above cases is preferable.

Furthermore, in the ion sensor catalyst of the present invention,the mixedoxide preferably containsamorphousruthenium oxide. This feature renders it possible to further achieve the following effect:
(1) As a result of the mixed oxide containing amorphous ruthenium oxide, the catalyst has an increased reactive surface area compared to the case where ruthenium oxide is entirely crystalline, so that oxidation current density for the same concentration of hydrogen phosphate ion increases, resulting in higher detection sensitivity and a wider quantifiable concentration range. Here, as has already been described, the difference in crystal structure as above can be relatively determined on the basis of the intensity of a diffraction line for ruthenium oxide obtained by X-ray diffractometry, and in the case of the mixed oxide containing amorphous ruthenium oxide, the intensity of a diffraction line for ruthenium oxide decreases. In the case where the ruthenium oxide contained in the mixed oxide is entirely amorphous, or the mixed oxide contains amorphous ruthenium oxide and tantalum oxide, the diffraction line for ruthenium oxide disappears.

Furthermore, the ion sensor of the present invention is an ion sensor for quantifying hydrogen phosphate ions in water, and includes a sensing electrode using the ion sensor catalyst described above. This feature renders it possible to achieve the following effects:
(1) The ion sensor catalyst, which contains a mixed oxide of ruthenium oxide and tantalum oxide, is used as the sensing electrode to determine hydrogen phosphate ion concentration on the basis of current flowing at the time of hydrogen phosphate ion oxidation, so that hydrogen phosphate ions can be selectively quantified in a short time with high detection sensitivity and accuracy over a wide concentration range.
(2) No large-sized device nor high power is needed to activate the ion sensor of the present invention, and the ion sensor can be miniaturized to a portable size, resulting in superior energy saving, compactness, and practicality.
(3) The ion sensor catalyst and other parts required for the ion sensor of the present invention can be produced at low cost, resulting in superior resource saving and mass producibility, and superior quantification stability and repeatability can be achieved in measuring hydrogen phosphate ion concentration.
(4) The ion sensor of the present invention does not cause other negative or positive ions, such as dihydrogen phosphate ion or chloride ion, to oxidize on the sensing electrode, and therefore can selectively detect only the hydrogen phosphate ions and determine the concentration thereof, resulting in superior measurement certainty and reliability.

Here, the ion sensor includes a detecting portion for detecting hydrogen phosphate ion oxidation current, and a control portion for applying a predetermined voltage between a sensing electrode and a counter electrode disposed in the detecting portion, or in the case where the sensing electrode and the counter electrode are provided in combination with a reference electrode, a predetermined potential to the sensing electrode with respect to the reference electrode, measuring oxidation current generated at the sensing electrode, calculating a final hydrogen phosphate ion concentration through arithmetic processing or suchlike, and displaying the calculation result. Note that the configuration and functionality of the control portion is not limited to the foregoing, and various features and functions used in existing chemical sensors and electrochemical sensors can be additionally provided. Moreover, the control portion requires at least the functions of: applying the predetermined voltage between the sensing electrode and the counter electrode, or in the case where the sensing electrode and the counter electrode are provided in combination with the reference electrode, the predetermined potential to the sensing electrode with respect to the reference electrode; and measuring the current generated at the sensing electrode, and as for other functions, various features and functions used in existing chemical sensors and electrochemical sensors can be additionally provided, as mentioned above. Accordingly, the configuration and functionality of the sensing portion in the ion sensor of the present invention will now be described further.

As has already been described, the sensing portion includes the sensing electrode and the counter electrode alone or in combination with the reference electrode. In any of these cases, the electrodes may be disposed individually, or may be integrally disposed on the same insulative substrate so as not to electrically contact one another. The material and the configuration of both the sensing electrode and the catalyst of the present invention are as have already been described, and the sensing electrode oxidizes hydrogen phosphate ions and conducts the resultant oxidation current to the control portion. The counter electrode causes some reduction reaction, and water reduction is generally known as reduction reaction caused in sample water. Various materials can be used as the material of the counter electrode, including platinum, silver, other metals, conductive compounds, such as conductive oxide, conductive plastic, and other materials used for electrochemical sensors and electrodes, and even the catalyst of the present invention can be used. However, as the material of the counter electrode, it is preferable to use a material which causes electrode reaction in which a reduction product does not rapidly react with hydrogen phosphate ions. For example, using platinum or silver causes water reduction as mentioned above.

In the configuration where the sensing electrode and the counter electrode are disposed in the sensing portion, the control portion applies an appropriate voltage between the sensing electrode and the counter electrode in order to cause hydrogen phosphate ion oxidation reaction at the sensing electrode. In this case, the waveform of the applied voltage may be constant or may change over time. Such a voltage wave form may be, for example, a rectangular voltage pulse or a waveform in which the voltage changes stepwise. In either case, the hydrogen phosphate ion oxidation current is desirably measured while a voltage which maximizes the value of the current is being applied. In the case where any coexisting ion present in sample water has an effect of inhibiting continuous hydrogen phosphate ion oxidation on the sensing electrode, for example, a voltage waveform which prevents such an inhibiting effect may be applied by inverting the polarity of the voltage applied between the sensing electrode and the counter electrode and thereby temporarily setting the voltage so as not to cause hydrogen phosphate ion oxidation at the sensing electrode. Such cyclical voltage inversion methods and necessary circuits are generally known in the field of electrochemistry such as electrochemical sensors, electroplating, industrial electrolysis, and batteries.

Next, in the configuration where the sensing electrode and the counter electrode in combination with the reference electrode are disposed in the sensing portion, the control portion applies an appropriate potential with respect to the reference electrode in order to cause hydrogen phosphate ion oxidation reaction at the sensing electrode. Note that by the terminology of electrochemistry, the difference in potential between two electrodes is generally referred to as the voltage, but in the case where the difference in potential with respect to a target electrode is defined using a standard electrode whose own potential does not change as in case of the reference electrode, such a difference is expressed as a potential rather than a voltage. As the reference electrode, for example, generally known reference electrodes, such as a silver-silver chloride electrode and a mercury-mercury oxide electrode, can be used. In the configuration where the sensing electrode and the counter electrode in combination with the reference electrode are disposed, the waveform of the applied potential may be constant or may change over time. Such a potential waveform may be, for example, a rectangular potential pulse or a waveform in which the potential changes stepwise. In either case, the hydrogen phosphate ion oxidation current is desirably measured while a potential which maximizes the value of the current is being applied. In the case where any coexisting ion present in sample water has an effect of inhibiting continuous hydrogen phosphate ion oxidation on the sensing electrode,for example,a potentialwaveform which prevents such an inhibiting effect may be applied by setting the potential of the sensing electrode with respect to the reference electrode lower than a natural potential (also referred to as an immersion potential) in sample water and thereby temporarily setting the potential so as not to cause hydrogen phosphate ion oxidation at the sensing electrode. Such cyclical potential changing methods and necessary circuits are generally known in the field of electrochemistry such as electrochemical sensors, electroplating, industrial electrolysis, and batteries.

In the case of detecting hydrogen phosphate ions using the sensing portion, for example, the sensing portion is immersed in sample water, or the sensing portion is brought into contact with sample water by dropping a predetermined amount of sample water on the sensing portion, thereby applying the voltage or potential and generating hydrogen phosphate ion oxidation current. Alternatively, oxidation current may be generated under the condition in which sample water is being delivered to the sensing portion by a flow injection method, or oxidation current may be generated by immersing the sensing portion in sample water within a container of a given capacity while causing the sample water to flow by stirring. Moreover, in these methods, there may be prepared a base liquid whose ingredients do not hinder hydrogen phosphate ion oxidation and whose electric conductivity has such a value as to render oxidation current measurable, and the base liquid may be sub jected to measurement after a predetermined amount of sample water is added thereto. Hydrogen phosphate ion concentration can be determined, for example, by the following methods: a calibration curve method in which, prior to the measurement of the sample water, hydrogen phosphate ion concentration in sample water is determined on the basis of the relationship between hydrogen phosphate ion concentration and oxidation current to be detected, which is obtained using two or three types of calibration liquids with known concentrations; and a standard addition method in which hydrogen phosphate ion concentration in sample water is obtained on the basis of concentrations of added hydrogen phosphate ions and oxidation current where a predetermined amount of calibration liquid with a known concentration is added to the sample water several times.

The ion sensor of the present invention is further provided with the function of measuring the pH and/or the temperature of sample water containing hydrogen phosphate ions. This configuration renders it possible to further achieve the following effects:
(1) The pH and/or the temperature of sample water containing hydrogen phosphate ions can be measured simultaneously with the hydrogen phosphate ions.
(2) As a result, characteristic values such as pH and temperature for sample water can be obtained simultaneously with hydrogen phosphate ion concentration.
(3) Further, it is possible to know whether there are any problems such as detection failure, which can be caused due to the pH and/or the temperature being different form their normal values for quantified hydrogen phosphate ion concentrations.

Furthermore, the quantification method of the present invention is a quantification method for determining the concentration of hydrogen phosphate ion or total phosphorus in water, and the quantification method includes a current measuring step for oxidizing hydrogen phosphate ions in the water and measuring the resultant oxidation current using the above-described ion sensor catalyst as a sensing electrode, and a concentration determining step for determining the concentration of hydrogen phosphate ion or total phosphorus on the basis of the oxidation current measured in the current measuring step. These features render it possible to achieve the following effects:
(1) By using the ion sensor catalyst including a mixed oxide of ruthenium oxide and tantalum oxide, hydrogen phosphate ion concentration can be selectively determined on the basis of current flowing at the time of hydrogen phosphate ion oxidation, so that the concentration of hydrogen phosphate ion or total phosphorus can be quantified in a short time with high detection sensitivity and accuracy over a wide concentration range.
(2) No large-sized device nor high power is needed to activate the ion sensor used in the quantification method of the present invention, so that the ion sensor can be miniaturized to a portable size, making it possible to perform quantification under various situations where the ion sensor is used on the spot or needs to be moved for measurement, resulting in superior versatility, easy handling, superior functionality, and superior mass producibility.
(3) The ion sensor catalyst and other parts required for the ion sensor used in the quantification method of the present invention can be provided at low cost, eliminating the need to use a specific solvent and reagent as in the conventional art, and thereby making it possible to perform highly stable and repeatable quantification at low cost, resulting in superior resource saving and practicality.
(4) The quantification method of the present invention does not cause other negative or positive ions, such as dihydrogen phosphate ion or chloride ion, to oxidize on the sensing electrode, and therefore can selectively detect only the hydrogen phosphate ion and determine the concentration thereof, resulting in superior measurement certainty and reliability.

Here, in the case where the concentration of hydrogen phosphate ion is determined, the pH of the sample water is adjusted to a level at which hydrogen phosphate ions can be present, or if the sample water itself is in such a pH range, the sample water is used without adjustment, hydrogen phosphate ions are oxidized by the sensing electrode using the catalyst of the present invention, and the concentration of hydrogen phosphate ion is determined on the basis of the resultant current. The sensing electrode required for the above, the configuration of the ion sensor including the sensing electrode, and the method for determining the concentration of hydrogen phosphate ions using the ion sensor are the same as described earlier. The sample water for which the present invention is intended is conceivably in the pH range of from neutral (pH = 7) to alkalescent, as is often the case with environmental water, and therefore, the concentration of hydrogen phosphate ion can be determined without pretreating the sample water. Moreover, the pH adjustment can be made readily, for example, by adding an alkaline compound or a solution thereof, or by using a buffer solution, but it is recommended not to use a phosphate buffer solution because the solution itself contains hydrogen phosphate ions. On the other hand, in the case where the concentration of total phosphorus is determined, organic phosphorus is subjected to ph adjustment after oxidation treatment, so that phosphorus is entirely converted to the form of hydrogen phosphate ions, as in the methods for determining the concentration of total phosphorus described in conjunction with the conventional art, and by using the resultant product as sample water, the concentration of hydrogen phosphate ion is determined in the aforementioned manner, and considered as the concentration of total phosphorus. The quantification method of the present invention renders it possible to determine the concentration of either hydrogen phosphate ion or total phosphorus by simply selecting whether or not to pretreat sample water.

In the quantification method of the present invention, a solution containing hydrogen phosphate ions is preferably caused to flow around the sensing electrode or delivered to the sensing electrode. This feature renders it possible to further achieve the following effects:
(1) By causing the solution containing hydrogen phosphate ions to flow around the sensing electrode or delivering the solution to the sensing electrode, convection and diffusion are promoted, so that oxidation current density for the same concentration of hydrogen phosphate ion increases when compared to the case where neither is performed, resulting in further increased detection sensitivity.
(2) Furthermore, oxidation reaction at the sensing electrode is promoted, resulting in shortened time to observe stable current, whereby the time required for measurement is further reduced.

Here, the solution containing hydrogen phosphate ions refers to not only sample water itself, such as environmental water or biological fluid, but also a test solution obtained by adding the sample water to a base liquid suitable for measurement. Such a base liquid is typically an aqueous solution, and one example, without limitation, is an aqueous solution of potassium chloride. However, the hydrogen phosphate ion has a buffering effect on the dihydrogen phosphate ion and the phosphate ion, and therefore, an aqueous solution containing dihydrogen phosphate ions or phosphate ions is not preferred as the base liquid. Moreover, the pH of the base liquid is preferably within the aforementioned pH range where hydrogen phosphate ions can be present (pH = 7 to 12), and therefore, it is not preferable to use a highly acidic or alkaline aqueous solution as the base liquid. Furthermore, as the solution containing hydrogen phosphate ions, a non-aqueous solvent, rather than an aqueous solution, can be used so long as the solvent has no effect of converting the form of hydrogen phosphate ions into another.

Furthermore, in the quantification method of the present invention, it is preferable that the solution containing hydrogen phosphate ions be delivered vertically to the sensing electrode. This feature renders it possible to further achieve the following effect:
(1) When compared to the case where the solution containing hydrogen phosphate ions is caused to flow around the sensing electrode or delivered parallel to the sensing electrode, convection and diffusion are further promoted, increasing oxidation current density for the same concentration of hydrogen phosphate ion, resulting in further increased detection sensitivity.

### EFFECT OF THE INVENTION

The present invention renders it possible to achieve the following effects:
1) A catalyst for detecting hydrogen phosphate ions can be synthesized by a generally known, simple method, and a sensing electrode for use with an ion sensor, which is shaped and sized to the application, use frequency, use environment, etc., of the ion sensor, and the ion sensor using such an electrode can be provided.
2) It is possible to provide the ion sensor in portable or carriable size, eliminating the need to bring sample water back to a laboratory for environmental analyses or suchlike, and thereby making it possible to perform immediate on-the-spot measurement and monitoring.
3) The present invention has the effect of allowing a low concentration of hydrogen phosphate ion or total phosphorus, or a high concentration of total phosphorus above emission standards, to be determined correctly in a short time compared to the conventional art.
4) The same ion sensor is capable of readily measuring the concentrations of both hydrogen phosphate ion and the total phosphorus.
5) When compared to the conventional art, the sensitivity to hydrogen phosphate ion is high, and quantification accuracy is high even over a wide concentration range, so that the concentration of hydrogen phosphate ion or total phosphorus can be measured correctly for various situations and purposes regardless of the measurer's skill in using the ion sensor.
6) Since the concentration of hydrogen phosphate ion can be determined in a short time, the time taken to measure hydrogen phosphate ions and total phosphorus in the environment or a living organism can be shortened, and the burden on the measurer during various types of analytical tasks or medical practice can be reduced, resulting in increased measurement efficiency.
7) The present invention allows quantification with a small amount of sample water, and eliminates the need for a specialized reagent or solvent for treating hydrogen phosphate ions, so that in quantifying hydrogen phosphate ions, effluents and wastewater, which cause environmental burdens, can be reduced.

### BRIEF DESCRIPTION OF THE DRAWINGS

FIG. 1 is a schematic diagram illustrating an example of an ion sensor using an ion sensor catalyst of the present invention.
FIG. 2 is a graph showing the relationship between hydrogen phosphate ion concentration andoxidation current density in Example 1.
FIG. 3 is a graph showing the relationship between the logarithm of hydrogen phosphate ion concentration andthe logarithm of oxidation current density in Example 1.
FIG. 4 is a graph showing the relationship between hydrogen phosphate ion concentration andoxidation current density in Example 2.
FIG. 5 is a graph showing the relationship between the logarithm of hydrogen phosphate ion concentration andthe logarithm of oxidation current density in Example 2.
FIG. 6 is a graph showing the relationship between hydrogen phosphate ion concentration andoxidation current density in Example 3.
FIG. 7 is a graph showing the relationship between the logarithm of hydrogen phosphate ion concentration andthe logarithm of oxidation current density in Example 3.
FIG. 8 is a graph showing the relationship between hydrogen phosphate ion concentration andoxidation current density in Example 4.
FIG. 9 is a graph showing the relationship between the logarithm of hydrogen phosphate ion concentration andthe logarithm of oxidation current density in Example 4.
FIG. 10 is a graph showing the relationship between hydrogen phosphate ion concentration andoxidation current density in Example 5.
FIG. 11 is a graph showing the relationship between the logarithm of hydrogen phosphateion concentration and the logarithm of oxidation current density in Example 5.
FIG. 12 is a graph showing the relationship between hydrogen phosphate ion concentration andoxidation current density in Example 6.
FIG. 13 is a graph showing the relationship between the logarithmofhydrogen phosphateion concentration andthe logarithm of oxidation current density in Example 6.

### MODES FOR CARRYING OUT THE INVENTION

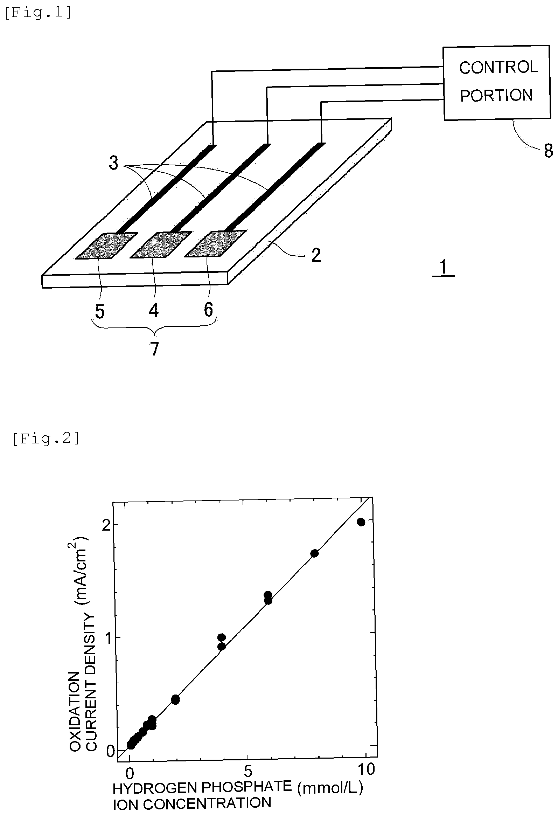

Hereinafter, an example of an ion sensor using an ion sensor catalyst according to the present invention will be described with reference to the drawings. It should be noted that the technical scope of the present invention is not limited to this example. FIG. 1 is a schematic diagram illustrating the ion sensor using the ion sensor catalyst according to the present invention. In FIG. 1, "1" indicates the ion sensor using the ion sensor catalyst according to the present invention, "2" indicates an insulative substrate made with any of various forms of insulative materials, including oxides such as alumina, carbides, nitrides, diamond, DLC (diamond-like carbon), and silicon, "3" indicates three conductive portions provided in the form of lines on the insulative substrate 2 using a conductive metal or alloy, any of various carbon materials (including conductive diamond, graphene, fullerene, etc.), conductive plastic, a compound such as conductive oxide, or a metal paste including silver or suchlike, "4" indicates a sensing electrode provided at one end of the middle conductive portion 3 by forming an ion sensor catalyst containing a mixed oxide of ruthenium oxide and tantalum oxide, "5" indicates a counter electrode provided at one end of another conductive portion 3 using various materials, including platinum, silver, other metals, conductive compounds, such as conductive oxide, conductive plastic, other materials used for electrochemical sensors and electrode materials, and even the catalyst of the present invention, "6" indicates a reference electrode provided at one end of the other conductive portion 3 using, for example, a silver-silver chloride electrode or a mercury-mercury oxide electrode, and "7" indicates a sensing portion, including the sensing electrode 4, the counter electrode 5, and the reference electrode 6. The ion sensor 1 includes a control portion 8, which applies a predetermined potential to the sensing electrode 4 with respect to the reference electrode 6, measures oxidation current generated at the sensing electrode 4, calculates a final hydrogen phosphate ion concentration through arithmetic processing, and displays the calculation result.

The usage of the ion sensor 1 thus configured will be described. The sensing electrode 4 oxidizes hydrogen phosphate ions, and conducts the resultant oxidation current through the conductive portion 3 to the control portion 8. The control portion 8 applies an appropriate potential with respect to the reference electrode 6 in order to cause hydrogen phosphate ion oxidation reaction at the sensing electrode 4. In the configuration where the sensing electrode 4, the counter electrode 5, and the reference electrode 6 are disposed, the waveform of the applied potential may be constant or may change over time. Such a potential waveform may be, for example, a rectangular potential pulse or a waveform in which the potential changes stepwise. In either case, the hydrogen phosphate ion oxidation current is desirably measured while a potential which maximizes the value of the current is being applied. In the case where any coexisting ion present in sample water has an effect of inhibiting continuous hydrogen phosphate ion oxidation on the sensing electrode 4, for example, a potential waveform which prevents such an inhibiting effect may be applied by setting the potential of the sensing electrode 4 with respect to the reference electrode 6 lower than a natural potential (also referred to as an immersion potential) in sample water and thereby temporarily setting the potential so as not to cause hydrogen phosphate ion oxidation at the sensing electrode 4.

In the case of detecting hydrogen phosphate ions using the sensingportion 7, for example, the sensingportion 7 is immersed in sample water, or the sensing portion 7 is brought into contact with sample water by dropping a predetermined amount of sample water on the sensing portion 7, thereby applying the voltage or potential and generating hydrogen phosphate ion oxidation current. Alternatively, oxidation current may be generated under the condition in which sample water is flowing by delivering the sample water to the sensing portion 7 by a flow injection method, or oxidation current may be generated by immersing the sensing portion 7 in sample water within a container of a given capacity while stirring the sample water. Moreover, in these methods, there may be prepared a base liquid whose ingredients do not hinder hydrogen phosphate ion oxidation and whose electric conductivity has such a value as to render oxidation current measurable, and the base liquid may be sub jected to measurement after a predetermined amount of sample water is added thereto. Hydrogen phosphate ion concentration can be determined, for example, by the following methods: a calibration curve method in which, prior to the measurement of the sample water, hydrogen phosphate ion concentration in sample water is determined on the basis of the relationship between hydrogen phosphate ion concentration and oxidation current to be detected, which is obtained using two or three types of calibration liquids with known concentrations; and a standard addition method in which hydrogen phosphate ion concentration in sample water is obtained on the basis of concentrations of added hydrogen phosphate ions and oxidation current where a predetermined amount of calibration liquid with a known concentration is added to the sample water several times.

The above example has been described with respect to the case where the sensing electrode 4 using the ion sensor catalyst, the counter electrode 5, and the reference electrode 6 are disposed on the same insulative substrate 2, but these electrodes may be disposed separately either on the insulative substrate or on a conductive substrate. For example, if the catalyst of the present invention is formed on a conductive substrate, the catalyst can be utilized as a sensing electrode on the conductive substrate serving as a conductive portion for external current output. It should be noted that the sensing portion 7 may be composed of the sensing electrode 4 and the counter electrode 5. In such a case, the control portion 8 applies an appropriate voltage between the sensing electrode 4 and the counter electrode 5 in order to cause hydrogen phosphate ion oxidation reaction at the sensing electrode 4.

The ion sensor 1 thus configured renders it possible to achieve the following effects:
(1) The ion sensor catalyst, which contains a mixed oxide of ruthenium oxide and tantalum oxide, is used as the sensing electrode to determine the concentration of hydrogen phosphate ion on the basis of current flowing at the time of hydrogen phosphate ion oxidation, so that hydrogen phosphate ions can be selectively quantified in a short time with high detection sensitivity and accuracy over a wide concentration range.
(2) No large-sized device nor high power is needed to activate the ion sensor of the present invention, and the ion sensor can be miniaturized to a portable size, resulting in superior energy saving, compactness, and practicality.
(3) The ion sensor catalyst and other parts required for the ion sensor of the present invention can be produced at low cost, resulting in superior resource saving and mass producibility; high catalytic activity is selectively exhibited for electrochemical oxidation of hydrogen phosphate ions in water over a wide range of hydrogen phosphate ion concentration while achieving high oxidation current density, so that concentration determination can be performed with accuracy regardless of the concentration range, and superior quantification stability and repeatability can be achieved in measuring hydrogen phosphate ion concentration.
(4) The ion sensor of the present invention does not cause other negative or positive ions, such as dihydrogen phosphate ion or chloride ion, to oxidize on the sensing electrode, and therefore can selectively detect only the hydrogen phosphate ion and determine the concentration thereof, resulting in superior measurement certainty and reliability.

The present invention will be described in detail below by way of Examples and Comparative Examples, but the present invention is not limited to Examples below.

### [Example 1]

A commercially available titanium plate (length: 5 cm, width: 1 cm, thickness: 1 mm) was immersed in a 10% oxalic acid solution at 90°C for 60 minutes and subjected to etching before washing in water and drying. Then, an application liquid was prepared by dissolving ruthenium(III) chloride trihydrate (RuCl₃· 3H₂O) and tantalum(V) chloride (TaCl₅) in a butanol (n-C₄H₉OH) solution containing 6 vol% concentrated hydrochloric acid, such that the molar ratio of ruthenium to tantalum was 80:20, and the total amount of ruthenium and tantalum was 70 g/L inmetal equivalent. The application liquid was applied to the dried titanium plate, dried at 120°C for 10 minutes, and then thermally decomposed for 20 minutes in an electric furnace maintained at 500°C. This process of application, drying, and thermal decomposition was repeated seven times in total to form a catalyst of Example 1 on the titanium plate, which is a conductive substrate, thereby producing a sensing electrode.

The catalyst of Example 1 was structurally analyzed by X-ray diffractometry, resulting in an X-ray diffraction image with a sharp diffraction peak corresponding to RuO₂ but no diffraction peak corresponding to Ta₂O₅. In addition, by an SEM (scanning electron microscope) and EDX (energy dispersive X-ray analysis), it was revealed that RuO₂ particles were dispersedly formed in a matrix phase of Ta₂O₅. From these results and analysis results for chemical states of ruthenium, tantalum, and oxygen by XPS (X-ray photoelectron spectroscopy), it was appreciated that the catalyst of Example 1 is a mixture of RuO₂ and Ta₂O₅. That is, in Example 1, the catalyst formed on the titanium plate is a mixed oxide of crystalline ruthenium oxide and amorphous tantalum oxide. A commercially available potassium chloride (KCl) reagent was dissolved in distilled water, thereby preparing a solution with a KCl concentration of 0.05 mmol/L, and a disodium hydrogen phosphate solution with a predetermined concentration, which was obtained by dissolving a disodium hydrogen phosphate (Na₂HPO₄) reagent in distilled water, was added in predetermined amounts, thereby preparing sample water with various hydrogen phosphate ion concentrations. Note that the pH of the sample water was from 8 to 9 over the hydrogen phosphate ion concentration range. The aforementioned sensing electrode was embedded in a polytetrafluoroethylene holder and disposed at a predetermined distance from a platinum plate serving as a counter electrode in the sample water within a beaker with the contact area of the electrode with the sample water being regulated to 1 cm². In addition, a saturated potassium chloride solution was placed in another container independently of the sample water, and a commercially available silver-silver chloride electrode was immersed in the solution as a reference electrode. The saturated potassium chloride solution and the sample water were connected using a salt bridge and a Luggin capillary, thereby producing a three-electrode electrochemical measuring cell. Note that the sample water was stirred by a stirrer during measurements.

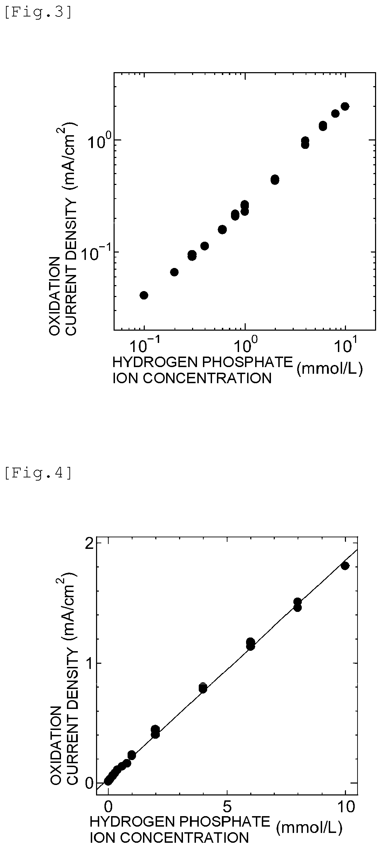

The three-electrode electrochemical measuring cell was used to perform cyclic voltammetry to measure current while scanning the potential of the sensing electrode with respect to the reference electrode at a constant speed, which revealed that in the case where the catalyst of Example 1 was used, an oxidation wave where oxidation current peaked near 0.95V was observed, revealing that the hydrogen phosphate ions were oxidized. Moreover, in the case of a potential higher than the peak, current increase due to oxygen generation caused by water electrolysis (i.e., decomposition of the entire sample water) was observed, but other than that, no current exhibiting oxidation or reduction was observed, and it was appreciated that hydrogen phosphate ions were selectively oxidized on the catalyst of Example 1. Next, the potential of the sensing electrode with respect to the reference electrode was set at 1.05V, and oxidation current density was measured for each concentration of sample water. Here, the oxidation current density (mA/cm²) is a value obtained by dividing an oxidation current (mA) flowing through the sensing electrode by the area of the electrode, but as described earlier, the area of the electrode of Example 1 is 1 cm², and therefore, in actuality, the oxidation current and the oxidation current density have the same value. Moreover, the oxidation current was obtained by subtracting a residual current flowing in the absence of hydrogen phosphate ions. An almost constant current density was exhibited in 10 to 20 seconds after the application of the potential to the sensing electrode. The relationship between oxidation current density and hydrogen phosphate ion concentration thus obtained is shown in FIGS. 2 and 3. In the case where the catalyst of Example 1 was used, the relationship obtained was proportional over a wide concentration range from low to high. Moreover, the detection sensitivity was obtained as 209 A·cm/mol on the basis of the slope of the concentration-oxidation current density line.

### [Example 2]

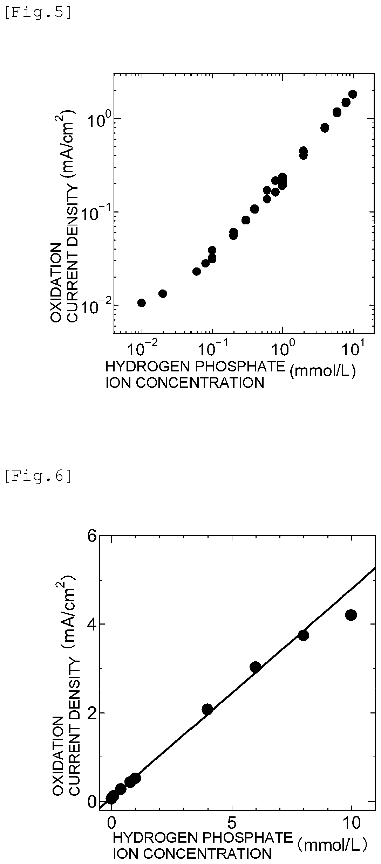

A sensing electrode was produced by forming a catalyst of Example 2 in the same manner as in Example 1, except that the molar ratio of ruthenium to tantalum in the application liquid was 30:70 in the method for forming the catalyst as described in Example 1. As a result of analyzing the catalyst, it was appreciated that the catalyst was made with a mixture of RuO₂ and Ta₂O₅, as in the case of Example 1. That is, in Example 2, as the catalyst, a mixed oxide composed of crystalline ruthenium oxide and amorphous tantalum oxide was formed on the titanium plate. As a result of performing cyclic voltammetry using the sensing electrode as in Example 1, hydrogen phosphate ion oxidation current peaked approximately at the same potential as in Example 1, but no oxidation current nor reduction current was observed, although a current for oxygen generation was observed, and therefore, it was appreciated that hydrogen phosphate ions were selectively oxidized on the catalyst of Example 2. Moreover, as in Example 1, oxidation current density in sample water in which hydrogen phosphate ions were dissolved in various concentrations was measured with the sensing electrode being maintained at a constant potential, with the result that an almost constant current density was exhibited in 10 to 20 seconds after the application of the potential to the sensing electrode. The relationship between oxidation current density and hydrogen phosphate ion concentration thus obtained is shown in FIGS. 4 and 5. In the case where the catalyst of Example 2 was used, the relationship obtained was proportional over a wide concentration range from low to high. Moreover, the detection sensitivity was obtained as 182 A·cm/mol on the basis of the slope of the concentration-oxidation current density line.

### [Comparative Example 1]

A commercially available titanium plate (length: 5 cm, width: 1 cm, thickness: 1 mm) was immersed in a 10% oxalic acid solution at 90°C for 60 minutes and subjected to etching before washing in water and drying. Then, an application liquid was prepared by dissolving hexachloroiridic acid hexahydrate (H₂IrCl₆• 6H₂O) in a butanol (n-C₄H₉OH) solution containing 6 vol% concentrated hydrochloric acid, such that the total amount was 70 g/L in metal equivalent. The application liquid was applied to the dried titanium plate, dried at 120°C for 10 minutes, and then thermally decomposed for 20 minutes in an electric furnace maintained at 470°C. This process of application, drying, and thermal decomposition was repeated seven times in total to form iridium dioxide on the titanium plate, which is a conductive substrate, thereby producing a sensing electrode. Note that the formed iridium dioxide was identified by X-ray diffractometry. As a result of performing cyclic voltammetry using the sensing electrode as in Example 1, hydrogen phosphate ion oxidation current peaked approximately at the same potential as in Example 1, but no oxidation current nor reduction current was observed, although a current for oxygen generation was observed, and therefore, it was appreciated that hydrogen phosphate ions were selectively oxidized on the iridium dioxide catalyst of Comparative Example 1. Moreover, as in Example 1, oxidation current density in sample water in which hydrogen phosphate ions were dissolved in various concentrations was measured with the sensing electrode being maintained at a constant potential, with the result that an almost constant current density was exhibited in 10 to 20 seconds after the application of the potential to the sensing electrode. Detection sensitivity was calculated on the basis of the relationship between oxidation current density and hydrogen phosphate ion concentration thus obtained, and was 0.30 times as much as that when the catalyst of Example 1 was used, and also 0.36 times as much as that when the catalyst of Example 2 was used.

There is no reported precedent for hydrogen phosphate ion oxidation by the catalyst of the present invention and the relationship between oxidation current density and concentration, but the results of Examples 1 and 2 revealed that oxidation current density and hydrogen phosphate ion concentration are in a proportional relationship over a wide concentration range from low to high and that the catalyst of the present invention functions as a catalyst for quantifying hydrogen phosphate ions. Moreover, it was also revealed that oxidation current density to be detected is approximately 1000 times as much as that in the conventional art where a composite oxide is used. In addition, from the above results, it was appreciated that in the case where the catalyst of the present invention is used, when compared to the conventional art where iridium dioxide is used, the detection sensitivity improves 2.8 to 3.3 times under the same measurement conditions over the same concentration range. Further, when comparing the detection sensitivity achieved by the catalyst of the present invention to the values (2.5 A·cm/mol to 85 A·cm/mol) found in Non-Patent Document 4, it was appreciated that in the case where the catalyst of the present invention is used, the detection sensitivity becomes up to 83 times higher.

### [Example 3]

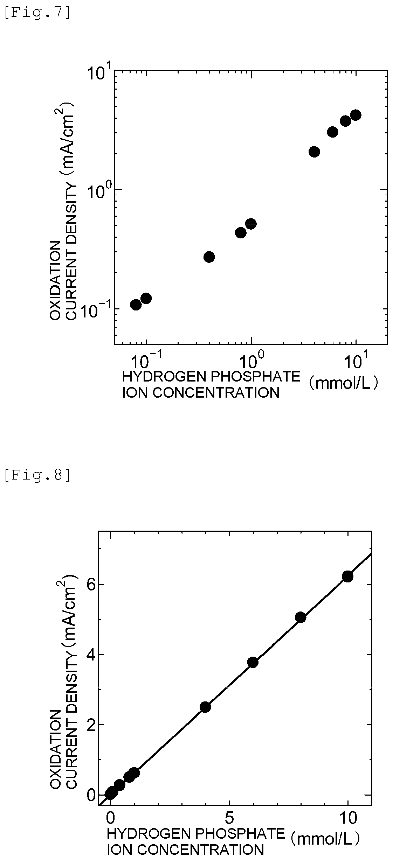

A sensing electrode was produced by forming a catalyst of Example 3 in the same manner as in Example 1, except that a titanium disk (diameter: 4 mm, thickness: 4 mm) was used in place of the titanium plate in the method for forming the catalyst as described in Example 1. As a result of analyzing the catalyst, it was appreciated that the catalyst was a mixture of crystalline RuO₂ and amorphous Ta₂O₅, as in the case of Example 1. That is, in Example 3, as the catalyst, a mixed oxide composed of crystalline ruthenium oxide and amorphous tantalum oxide, as in Example 1, was formed on the titanium disk. As a result of performing cyclic voltammetry using the sensing electrode as in Example 1, hydrogen phosphate ion oxidation current peaked approximately at the same potential as in Example 1, but no oxidation current nor reduction current was observed, although a current for oxygen generation was observed, and therefore, it was appreciated that hydrogen phosphate ions were selectively oxidized on the catalyst of Example 3. Moreover, as in Example 1, oxidation current density in sample water in which hydrogen phosphate ions were dissolved in various concentrations was measured with the sensing electrode being maintained at a constant potential, but in Example 3, the sensing electrode was being rotated during the measurements using a rotating disk electrode device, while in Example 1, the sample water in the beaker was being stirred by a stirrer during the measurements. The configuration of the electrochemical measuring cell was the same as in Example 1, except that the sensing electrode was rotated using the rotating disk electrode device. As a result, in Example 3, by the rotation of the sensing electrode, the sample water was delivered to the sensing electrode from the vertical direction to the sensing electrode, while in Example 1, the sample water flows in a direction parallel to the sensing electrode. Note that other than the approach that rotates the sensing electrode, the delivery can be performed as well by a device, as used in a flow injection method or suchlike, which causes sample water to flow toward the stationary sensing electrode from the vertical direction to the sensing electrode. The relationship between oxidation current density and hydrogen phosphate ion concentration thus obtained is shown in FIGS. 6 and 7. Consequently, the relationship obtained was proportional over a wide concentration range from low to high. Moreover, the detection sensitivity was obtained as 471 A·cm/mol on the basis of the slope of the concentration-oxidation current density line. That is, in Example 3, by changing the manner of supplying the sample water, the detection sensitivity increases 2.2 times as much as that in Example 1, even when the same catalyst is used.

### [Example 4]

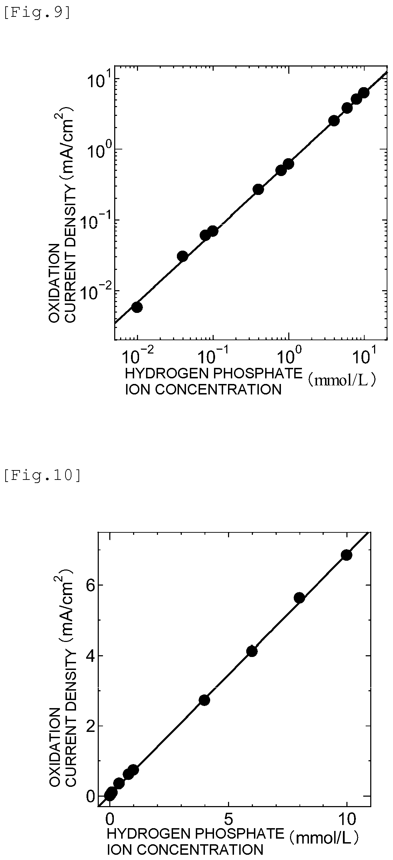

A sensing electrode was produced by forming a catalyst of Example 4 in the same manner as in Example 1, except that a titanium disk (diameter: 4 mm, thickness: 4 mm) was used in place of the titanium plate and thermally decomposed for 20 minutes in an electric furnace maintained at 260°C in the method for forming the catalyst as described in Example 1. As a result of analyzing the catalyst, it was appreciated that in Example 4, the catalyst was made with a mixture of amorphous RuO₂ and amorphous Ta₂O₅, while in Example 1, the catalyst was a mixture of crystalline RuO₂ and amorphous Ta₂O₅. That is, in Example 4, as the catalyst, a mixed oxide composed of amorphous ruthenium oxide and amorphous tantalum oxide was formed on the titanium disk. As a result of performing cyclic voltammetry using the sensing electrode as in Example 1, hydrogen phosphate ion oxidation current peaked approximately at the same potential as in Example 1, but no oxidation current nor reduction current was observed, although a current for oxygen generation was observed, and therefore, it was appreciated that hydrogen phosphate ions were selectively oxidized on the catalyst of Example 4. Moreover, as in Example 3, oxidation current density in sample water in which hydrogen phosphate ions were dissolved in various concentrations was measured with the sensing electrode being maintained at a constant potential, with the result that an almost constant current density was exhibited in 10 to 20 seconds after the application of the potential to the sensing electrode. The relationship between oxidation current density and hydrogen phosphate ion concentration thus obtained is shown in FIGS. 8 and 9. Consequently, the relationship obtained was proportional over a wide concentration range from low to high. Moreover, the detection sensitivity was obtained as 624 A·cm/mol on the basis of the slope of the concentration-oxidation current density line. That is, in Example 4, since the catalyst ruthenium oxide of the catalyst is amorphous, the detection sensitivity increases 1.3 times as much as that in Example 3 where the catalyst has the same composition ratio of ruthenium to tantalum. Moreover, since the ruthenium oxide is amorphous, and the delivery direction of sample water is vertical to the sensing electrode, the detection sensitivity increases 3 times as much as that in Example 1. Furthermore, it was appreciated that in Example 4, a straight line was plotted on either a linear scale (FIG. 8) or a double-logarithmic scale (FIG. 9), and the detection sensitivity was invariable over a wide three-digit concentration range of from 10⁻² mmol/L to 10 mmol/L. More specifically, since the ruthenium oxide of the catalyst is amorphous, the detection sensitivity increases when compared to Example 3, and further, the concentration range that can be quantified expands, revealing that hydrogen phosphate ions can be quantified with high detection sensitivity over any concentration range.

### [Example 5]

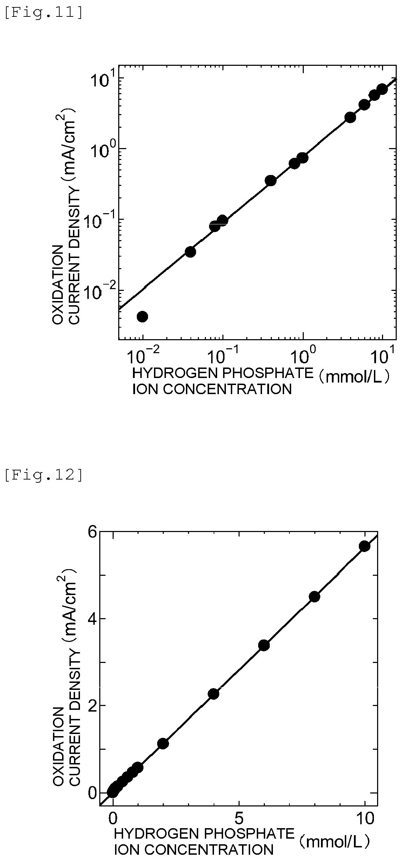

A sensing electrode was produced by forming a catalyst of Example 5 in the same manner as in Example 4, except that the molar ratio of ruthenium to tantalum in the application liquid was 50:50 in the method for forming the catalyst as described in Example 4. As a result of analyzing the catalyst, it was appreciated that the catalyst was made with a mixture of amorphous RuO₂ and amorphous Ta₂O₅, as in Example 4. That is, in Example 5 also, as the catalyst, a mixed oxide composed of amorphous ruthenium oxide and amorphous tantalum oxide was formed on the titanium disk. As a result of performing cyclic voltammetry using the sensing electrode as in Example 1, hydrogen phosphate ion oxidation current peaked approximately at the same potential as in Example 1, but no oxidation current nor reduction current was observed, although a current for oxygen generation was observed, and therefore, it was appreciated that hydrogen phosphate ions were selectively oxidized on the catalyst of Example 5. Moreover, as in Example 4, oxidation current density in sample water in which hydrogen phosphate ions were dissolved in various concentrations was measured with the sensing electrode being maintained at a constant potential, with the result that an almost constant current density was exhibited in 10 to 20 seconds after the application of the potential to the sensing electrode. The relationship between oxidation current density and hydrogen phosphate ion concentration thus obtained is shown in FIGS. 10 and 11. Consequently, the relationship obtained was proportional over a wide concentration range from low to high. Moreover, the detection sensitivity was obtained as 685 A·cm/mol on the basis of the slope of the concentration-oxidation current density line, i.e., higher than in the case of the catalyst of Example 4. Moreover, since the ruthenium oxide is amorphous, and the delivery direction of sample water is vertical to the sensing electrode, the detection sensitivity increases 3.3 times as much as that in Example 1. Furthermore, it was appreciated that in Example 5, a straight line was plotted on either a linear scale (FIG. 10) or a double-logarithmic scale (FIG. 11), and the detection sensitivity was invariable over a wide three-digit concentration range of from 4×10⁻² mmol/L to 10 mmol/L. More specifically, it was revealed that hydrogen phosphate ions can be quantified with high detection sensitivity over any concentration range.

### [Example 6]

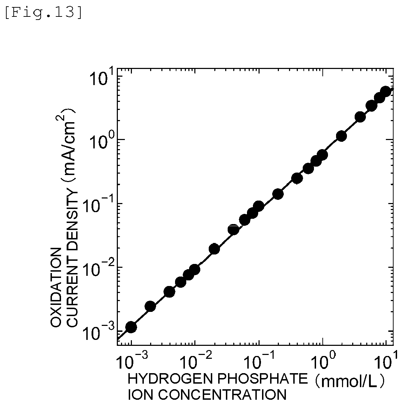

A sensing electrode was produced by forming a catalyst of Example 6 in the same manner as in Example 4. Moreover, as in Example 4, oxidation current density in sample water in which hydrogen phosphate ions were dissolved in various concentrations was measured with the sensing electrode being maintained at a constant potential. However, prior to the measurement, the potential was applied and maintained for 5 minutes, and disodium hydrogen phosphate was added after residual current attenuated sufficiently. An almost constant current density was exhibited in about 20 seconds after the addition of the disodium hydrogen phosphate. The relationship between oxidation current density and hydrogen phosphate ion concentration thus obtained is shown in FIGS. 12 and 13. Consequently, the relationship obtained was proportional over a wide concentration range from low to high. Moreover, the detection sensitivity was obtained as 562 A·cm/mol on the basis of the slope of the concentration-oxidation current density line. Furthermore, it was appreciated that in Example 6, a straight line was plotted on either a linear scale (FIG. 12) or a double-logarithmic scale (FIG. 13), and the detection sensitivity was invariable over a wide four-digit concentration range of from 10⁻³ mmol/L to 10 mmol/L. More specifically, it was revealed that by keeping the residual current sufficiently low during the measurement, it is rendered possible to perform quantification over a wider concentration range than in Example 4, and at the same time, hydrogen phosphate ions can be quantified with high detection sensitivity over any concentration range.

### INDUSTRIAL APPLICABILITY

The present invention provides an inexpensive ion sensor catalyst capable of detecting hydrogen phosphate ions in water and determining hydrogen phosphate ion concentration on the basis of oxidation current density for the ions, the ion sensor catalyst being characterized by providing higher detection sensitivity than conventional ion sensor catalysts, capable of maintaining high detection sensitivity over a wide range of hydrogen phosphate ion concentration, achieving a proportional relationship between concentration and oxidation current density, so that the concentration can be determined with accuracy regardless of the concentration range, and allowing oxidation current to reach a steady-state value in a short time, so that time to determine current density is short, resulting in shortened time to quantify hydrogen phosphate ions, and stable response to hydrogen phosphate ion oxidation can be made repeatedly; the present invention also provides an ion sensor for hydrogen phosphate ion and a quantification method, the ion sensor is capable of quantifying hydrogen phosphate ions in a short time with high detection sensitivity and accuracy over a wide concentration range by using the catalyst, while being provided in a portable size, mass-produced at low cost, and at the same time, being superior in measurement stability and repeatability, and the quantification method uses the sensor and achieves highly sensitive and repeatable quantification of total phosphorus and hydrogen phosphate ions, thus the present invention contributes to enhance work efficiency, reliability, etc., in measurement and monitoring of total phosphorus and hydrogen phosphate ions in a wide range of applications such as environmental measurement, medical practice, and various analytical tasks.

### DESCRIPTION OF THE REFERENCE CHARACTERS

- 1: ion sensor
- 2: insulative substrate
- 3: conductive portion
- 4: sensing electrode
- 5: counter electrode
- 6: reference electrode
- 7: sensing portion
- 8: control portion

The present specification also includes the subject matter of the following clauses:
1. An ion sensor catalyst for electrochemically oxidizing hydrogen phosphate ions in water, including a mixed oxide of ruthenium oxide and tantalum oxide.
2. The ion sensor catalyst according to clause 1, wherein the mixed oxide contains ruthenium and tantalum in a molar ratio of from 30:70 to 80:20.
3. The ion sensor catalyst according to clause 1 or 2, wherein the mixed oxide contains amorphous ruthenium oxide.
4. An ion sensor for quantifying hydrogen phosphate ions in water, comprising a sensing electrode using an ion sensor catalyst of any one of clauses 1 through 3.
5. The ion sensor according to clause 4, further comprising:
   a detecting portion for detecting hydrogen phosphate ion oxidation current by the sensing electrode; and
   a control portion for calculating a concentration of the hydrogen phosphate ions on the basis of the oxidation current detected by the detecting portion and displaying the calculation result, the control portion being electrically connected to the detecting portion.
6. The ion sensor according to clause 5, wherein the detecting portion includes the sensing electrode and a counter electrode alone or in combination with a reference electrode.
7. The ion sensor according to clause 6, further comprising one insulative substrate, wherein the sensing electrode and the counter electrode in the detecting portion, alone or in combination with the reference electrode, are formed on the insulative substrate.
8. The ion sensor according to any one of clauses 4 through 7, having a function of measuring a pH and/or a temperature of sample water containing hydrogen phosphate ions.
9. A quantification method for determining a concentration of hydrogen phosphate ion and/or total phosphorus in water, comprising:
   a current measuring step for oxidizing hydrogen phosphate ions in the water and measuring the resultant oxidation current using an ion sensor catalyst of any one of clauses 1 through 3 as a sensing electrode; and
   a concentration determining step for determining the concentration of hydrogen phosphate ion or total phosphorus on the basis of the oxidation current measured in the current measuring step.
10. The quantification method according to clause 9, wherein in the current measuring step, a solution containing hydrogen phosphate ions is caused to flow around the sensing electrode or delivered to the sensing electrode.
11. The quantification method according to clause 10, wherein in the current measuring step, the solution containing hydrogen phosphate ions is delivered vertically to the sensing electrode.

## Claims

1. An ion sensor for quantifying hydrogen phosphate ions in water, comprising a sensing electrode using an ion sensor catalyst, the ion sensor catalyst including a mixed oxide of ruthenium oxide and tantalum oxide.

2. The ion sensor according to claim 1, wherein the mixed oxide contains ruthenium and tantalum in a molar ratio of from 30:70 to 80:20.

3. The ion sensor according to claim 1 or 2, wherein the mixed oxide contains amorphous ruthenium oxide.

4. The ion sensor according to any one of claims 1 through 3, further comprising:
a detecting portion for detecting hydrogen phosphate ion oxidation current by the sensing electrode; and
a control portion for calculating a concentration of the hydrogen phosphate ions on the basis of the oxidation current detected by the detecting portion and displaying the calculation result, the control portion being electrically connected to the detecting portion.

5. The ion sensor according to claim 4, wherein the detecting portion includes the sensing electrode and a counter electrode alone or in combination with a reference electrode.

6. The ion sensor according to claim 5, further comprising one insulative substrate, wherein the sensing electrode and the counter electrode in the detecting portion, alone or in combination with the reference electrode, are formed on the insulative substrate.
